(19) Europäisches Patentamt
European Patent Office
Office européen des brevets

(11) EP 0 636 368 B1

(12) EUROPEAN PATENT SPECIFICATION

(45) Date of publication and mention of the grant of the patent:
**10.12.1997 Bulletin 1997/50**

(51) Int. Cl.$^6$: **A61K 31/15**, A61K 31/495

(21) Application number: **94111107.2**

(22) Date of filing: **18.07.1994**

# (54) The use of 2- phenylmethylene -1- 3'-(amino)-2'-(hydroxy)--propoxy-imino -cyclohexane derivatives

Die Verwendung von 2-Phenylmethylene-1-3'-(amino)-2-(hydroxy)-propoxy-imino-cyclohexane-Derivaten

L'utilisation de dérivés de 2-phénylméthylène-1-3'-(amino)-2-(hydroxy)-propoxy-imino-cyclohexane

(84) Designated Contracting States:
**AT BE CH DE DK FR GB GR LI NL PT SE**
Designated Extension States:
**SI**

(30) Priority: **20.07.1993 HU 9302081**

(43) Date of publication of application:
**01.02.1995 Bulletin 1995/05**

(73) Proprietor:
**EGIS GYOGYSZERGYAR RT.**
**1106 Budapest (HU)**

(72) Inventors:
- **Szirt, geb. Kıszelly, Enikö**
  **H-1105 Budapest (HU)**
- **Budai, Zoltan, Dr.**
  **H-1023 Budapest (HU)**
- **Mezei, Tibor, Dr..**
  **H-1221 Budapest (HU)**
- **Blasko, Gabor, Dr.**
  **H-1113 Budapest (HU)**
- **Kazo, geb. Daroczi, Klara**
  **H-1161 Budapest (HU)**
- **Egyed, Andras, Dr.**
  **H-1145 Budapest (HU)**
- **Gigler, Gabor**
  **H-1067 Budapest (HU)**
- **Fekete, Marton, Dr.**
  **H-1027 Budapest (HU)**
- **Reiter, geb. Esses, Klara, Dr.**
  **H-1022 Budapest (HU)**
- **Simig, Gyula, Dr.**
  **H-1126 Budapest (HU)**
- **Szemerédi, Katalin, Dr.**
  **H-1035 Budapest (HU)**

(74) Representative:
**Beszédes, Stephan G., Dr.**
**Patentanwalt**
**Postfach 11 68**
**85201 Dachau (DE)**

(56) References cited:
**EP-A- 0 168 245** **GB-A- 2 235 198**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.15.3/3.4

## Description

The invention is concerned with a new use of 2-[phenylmethylene]-1-[3'-(amino)-2'-(hydroxy)-propoxy-imino]-cyclohexane derivatives.

It is known that ketone derivatives of the formula

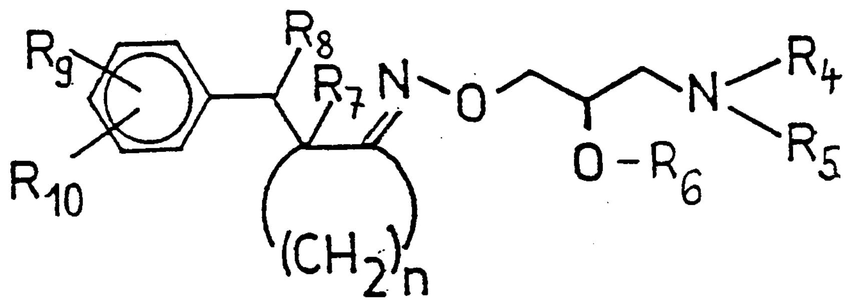

VI ,

wherein

| | |
|---|---|
| $R_9$ and $R_{10}$ | independently stand for hydrogen atom(s), halogen atom(s), lower alkoxy group(s) or |
| $R_9$ and $R_{10}$ | together form a methylenedioxy group, |
| $R_7$ and $R_8$ | both represent hydrogen or |
| $R_7$ and $R_8$ | together form a chemical bond, |
| $R_4$ and $R_5$ | independently from each other mean hydrogen atom(s), straight or branched chain saturated or unsaturated $C_{1-12}$ alkyl group(a), optionally substituted by a dialkylaminoalkyl, dimethoxyphenyl or phenyl group, or $C_{3-7}$ cycloalkyl group(s) or |
| $R_4$ and $R_5$ | together with the nitrogen atom to which they are attached form a 4 to 7 membered heterocyclic group comprising optionally an additional oxygen, sulfur or nitrogen atom and the additional nitrogen atom optionally may bear a phenyl, benzyl or $C_{1-3}$ alkyl group, and optionally the latter substituents may be substituted by a hydroxyl group or 1 or 2 methoxy group(s), halogen atom(s) or trifluoromethyl group(s) or |
| $R_4$ and $R_5$ | together with the nitrogen atom to which they are attached form a piperidine ring optionally containing a double bond and optionally substituted by a phenyl or benzyl group, |
| $R_6$ | stands for a hydrogen atom or a benzoyl group and |
| n | is 3, 4, 5 or 6, |

possess antianginal and/or antiarrhythmic, gastric secretion inhibiting, local anaesthetic, tranquillo-sedative, antiinflammatory, analgetic and, in some cases, calcium antagonist effects (published GB Patent application 2,235,198) the gastric secretion inhibiting effect having been indicated only for the compounds R,S-2-(E)-phenylmeteylene-1-(E)-[3-(4-methyl-1-piperazinyl)-2'-hydroxy-propoxyimino]-cyclohexane {Example 4}, R,S-2-(E)-phenylmethylene-1-(E)-{3-[bis-(1-methylethyl)-amino]-2-hydroxy-propoxyimino}-cyclohexane ⟨Example 1⟩, R,S-2-(E)-(4-chlorophenyl-methylene)-1-(E)-(3-butylamino-2-hydroxy-propoxyimino)-cyclohexane {Example 29}, R,S-2-(E)-(4-chloro-phenyl-methylene)-1-(E)-[3-(1,1-dimethylpropin-2-yl-amino)-2-hydroxy-propoxyimino]-cyclohexane {Example 32}, R,S-2-(E)-phenylmethylene-1-(E)-(3-morpbolino-2-hydroxy-propoxyimino)-cyclooctane {Example 18}, R,S-2-(E)-(4-chlorophenyl-methylene)-1-(E)-[3-(1-methylethyl-amino)-2'-hydroxy-propoxyimino]-cyclohexane {Example 26}, R,S-2-(E)-(3,4-dichlorophenyl-methylene)-1-(E)-(3-butylamino-2-hydroxy-propoxyimino)-cyclohexane {Example 33}, R,S-2-(E)-(4'-methoxyphenyl-methylene)-1-(E)-(3-propylamino-2-hydroxy-propoxyimino)-cycloheptane {Example 43}, R,S-2-(E)--phenylmethylene-1-(E)-[3-(3-dimethylamino-1-propylamino)-2- hydroxy-propoxyimino]-cyclohexane {Example 11}, R,S-2-(E)-phenylmethylene-1-(E)-{[4-(2-hydroxyethyl)-1-piperazinyl]-2-hydroxy-propoxyimino}-cyclohexane ⟨Example 13⟩, R,S-2-(E)-(4-chlorophenyl-methylene)-1-(E)-[3'-(N-methyl-N-cyclohexylamino)-2-hydroxy-propoxyimino]-cyclohexane {Example 45}, R,S-2-(E)-(4-methoxyphenyl-methylene)-1-(E)-[3-(3-dimethylamino-1-propylamino)-2-hydroxy-propoximino]-cyclohexane {Example 41}, R,S-2-(E)-(4-methoxyphenyl-methylene)-1-(E)-(3-cyclopropylamino-2-hydroxy-propoxyimino)-cyclohexane {Example 40} and R,S-2-(E)-phenylmethylene-1-(E)-[3-(1-methylethyl-amino)-2-hydroxy-propoxyimino]-cyclooctane {Example 48} and no antimicrobial activity effect having been indicated at all.

From published European Patent application 168 245 compounds of general formula

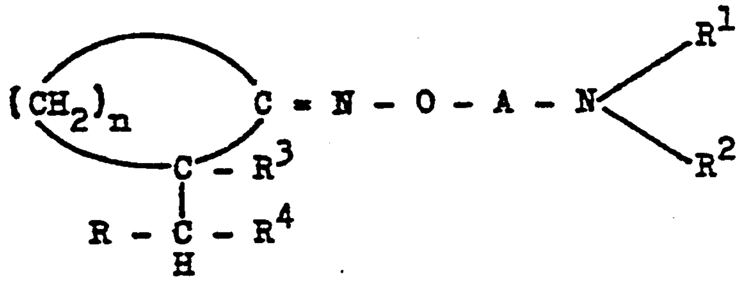

VII,

wherein
if n = 4, then

$A_1$)

$R^3$ end $R^4$ together form a chemical bond,
R is phenyl,
A means trimethylene,
$R^1$ and $R^2$ represent a methyl group each and
the molecule has an (E,Z) configuration; or

$A_2$)

$R^3$ and $R^4$ together form a chemical bond,
R means 4-chlorophenyl,
A is ethylene,
$R^1$ is isopropyl,
$R^2$ is hydrogen and
the molecule has an (E,E) configuration; or

$A_3$)

$R^3$ and $R^4$ together form a chemical bond,
R is 4-chlorophenyl,
A is trimethylene,
$R^1$ and $R^2$ represent an isopropyl group each and
the molecule has an (E,E) configuration; or

$A_4$)

$R^3$ and $R^4$ together form a chemical bond,
R means phenyl,
A is trimethylene,
$R^1$ and $R^2$ represent an isopropyl group each and
the molecule has an (E,E) configuration; or

$A_5$)

$R^3$ and $R^4$ together form a chemical bond,
R is phenyl,
A means ethylene,
$R^1$ stands for isopropyl,
$R^2$ is hydrogen and
the molecule has an (E,E) configuration; or

$A_6$)

$R^3$ and $R^4$ together form a chemical bond,
R means phenyl,
A is ethylene,
$R^1$ and $R^2$ stand for an isopropyl group each and
the molecule has an (E,z) configuration; or

if n = 5, then

$B_1$)

$R^3$ and $R^4$ together form a chemical bond,
R is phenyl,
A is ethylene,
$R^1$ and $R^2$ represent a methyl group each and
the molecule has a (Z,E) configuration; or

$B_2$)

$R^3$ and $R^4$ stand for hydrogen each,
R means phenyl,
A is ethylene,
$R^1$ and $R^2$ stand for a methyl group each and
the molecule has an (E,E) configuration; or

$B_3$)

$R^3$ and $R^4$ together form a chemical bond,
R is phenyl,
A stands for ethylene,
$R^1$ and $R^2$ represent a methyl group each and
the molecule has an (E,E) configuration; or

$B_4$)

$R^3$ and $R^4$ together form a chemical bond,
R means phenyl,
A is ethylene,
$R^1$ and $R^2$ represent an isopropyl group each
and the molecule has a (Z,E) configuration;

or if n = 6, then

C)

$R^3$ and $R^4$ together form a chemical bond,
R is phenyl,
A stands for trimethylene,
$R^1$ and $R^2$ represent a methyl group each and
the molecule has an (E,E) configuration,

and their pharmaceutically acceptable acid addition salts and their antianginal effect has been known.

Recently, gastric and duodenal ulcers are among the most widespread diseases that are accompanied by a continuously increasing number of oesophagitis reflux cases. It is more and more believed by clinical practitioners that the most adequate method for the treatment of peptic ulcer consists in killing the Helicobacter pylori bacteria being present in the organism of the patient. The antiulcer use of N-[2-{5-(dimethylaminomethyl)-furfurylthio}-ethyl]-N'-[methyl]-2-[nitro]-1,1-ethendiamine 〈8-{5-(dimethylaminomethyl)-2-furyl}-3-{nitromethylene}-7-{thia}-2,4-di-{aza}-octane〉 [ranitidine] and 1-[cyano]-2-[methyl]-3-[2-{(5-〈methyl〉-4-imidazoyl)-methylthio}-ethyl]-guanidine [cimetidine] having an otherwise favourable histamine $H_2$ receptor antagonist effect was highly diminished by the discovery that the killing of Helicobacter pylori prevents the relapse of ulcer. As a consequence, the use of 5-[methoxy]-2-[{4-[methoxy)-3,5-di-(methyl)-2-pyridyl}-methyl sulfinyl]-benzimidazole [omeprazole] inhibiting the $H^+/K^+$-ATP-ase enzyme becomes more and more widespread (this mechanism of action is quite novel compared to those known in the art). Its spreading is significantly promoted by the safety of its therapeutical use and the low number of side effects.

Compounds inhibiting the proton pump i.e. being inhibitors of $H^+/K^+$-ATP-ase are not only preferred and more rapidly acting compounds than the $H_2$ antagonists, but, in comparison to $H_2$ antagonists, they are effective also in the treatment of oesophagitis reflux, which is becoming more and more widespread. A common drawback of the antiulcer drugs possessing various mechanisms is that they do not respresent an effective therapeutical method against Helicobacter pylori bacteria. Thus, at present, 5-[methoxy]-2-[{4-(methoxy)-3,5-di-(methyl)-2-pyridyl}-methylsulfinyl]-benzimidazole [omeprazole] is combined with α-amino-4-hydroxybenzyl-penicillin ⟨(2S,5R,6R)-6-[(R)-(-)-2-(amino)-2-(p-hydroxyphenyl)-acetamido]-3,3-di-{methyl}-7-{oxo}-4-{thia}-1-{aza}-bicyclo[3.2.0]-heptane-2-carboxylic acid⟩ [amoxycillin] having high antibacterial activity to obtain better therapeutical result. Previously, the combination of colloidal bismuth and 1-[2-(hydroxy)-ethyl]-2-[methyl]-5-[nitro]-imidazole ⟨2-(methyl)-5-(nitro)-imidazole-1-ethanol⟩ [metronidazole] with α-amino-4-hydroxybenzyl-penicillin [amoxycillin] had been used, but 1-[2-(hydroxy)-ethyl]-2-[methyl]-5-[nitro]-imidazole [metronidazole] caused a great problem since, in addition to its rather unfavourable side effects, bacteria became increasingly resistant to it.

As an inhibitor of the proton pump, 5-[methoxy]-2-[{4-(methoxy)-3,5-di-(methyl)-2-pyridyl}-methylsulfinyl]-benzimidazole [omeprazole] plays some role in the prevention of ulcer development caused by non-steroidal antiinflammatory drugs. At present, 3-[4-{trans-4-(aminomethyl)-cyclohexylcarbonyloxy}-phenyl]-propionic acid [cetraxate] and the basic aluminum salt of saccharose hydrogen sulfate ⟨the basic aluminum salt of β-D-fructofuranosyl α-D-glucopyranoside hydrogen sulfate⟩ [sucralfate] are the most frequently used ulcer preventing and cytoprotective agents.

Thus, there is a demand on the use of active principles simultaneously exerting:

a) a rapid ulcer healing effect,
b) a prophylactic effect against ulcer formation induced by non-steroidal antiinflammatory drugs and
c) an effect of completely killing the Helicobacter pylori bacteria without the risk of intolerable side effects.

Hence the problem underlying to the invention is to create the use of compounds having the above effects for preparing medicaments having gastroprotective and antimicrobial including antiulcer activity.

This surprisingly has been attained by the present invention.

It was found that the compounds as defined in the following falling within the scope of formula VI possess valuable gastroprotective and antimicrobial activity, said gastroprotective activity being independent of the inhibition of the gastric acid secretion.

Consequently, the above defined 2-[phenylmethylene]-1-[3'-(amino)-2'-(hydroxy)-propoxy-imino]-cyclohexane derivatives can be utilized as active principles for preparing medicaments useful for the prevention and/or treatment of gastroic diseases including ulcer.

Hence the subject-matter of the invention is the use of 2-[phenylmethylene]-1-[3'-(amino)-2'-(hydroxy)-propoxy-imino]-cyclohexane derivatives of formula

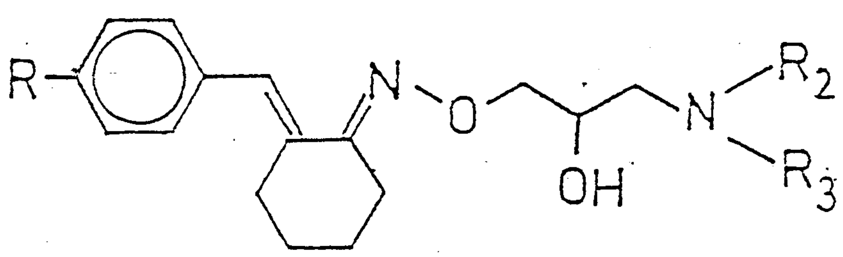

I ,

wherein

R means a hydrogen or halogen atom,

$R_2$ represents a straight chain $C_{1-8}$ alkyl group and

$R_3$ means a hydrogen atom, a straight chain $C_{1-8}$ alkyl group or a $C_{3-7}$ cycloalkyl group,

and their stereoisomers and optical isomers and mixtures thereof as well as pharmaceutically acceptable acid addition salts and quaternary ammonium derivatives thereof, optionally in admixture with 1 or more carrier(s) commonly used in

pharmaceutical compositions, for preparing medicaments having combined gastroprotective and antimicrobial including antiulcer activity.

The compound of formula I comprise chiral carbon atoms as well as double bonds, thus stereoismers and optimal isomers may exist. The use according to the invention comprises that of such stereoisomers or optical isomers or mixtures thereof.

In the present text "cytoprotection" is defined as the ability of certain compounds to protect the organs of the digestive system against deteriorative effects. In case of the gastrointestinal system, "cytoprotection" is also called "gastroprotection". Therefore, in the present text the expressions "cytoprotection", "cytoprotective" and "gastroprotection" and "gastroprotective", respectively, are used as synonyms.

The use according to the invention fundamentally differs from that according to published GB Patent application 2,235,198 in that the range of compounds above defined is suitable for preparing medicaments having antimicrobial activity, particularly against Helicobacter pylori, whereas in the said publication for no compound such an activity has been indicated. As regards the use for preparing medicaments having gastroprotective activity the present invention differs from published GB Patent application 2,235,198 in that as far as compounds within the above definition used according to the invention described in published GB Patent application 2,235,198 or most of them not even a gastric secretion inhibiting activity has been indicated in which regard it is to be noted that anyhow the gastroprotective activity of the compounds used according to the invention is surprisingly independent of the inhibition of the gastric acid secretion.

The use according to the invention is fundamentally different from that according to published European Patent application 168,245 in that it is for preparing medicaments having gastroprotective and antimicrobial activity as proposed to the indication only of an antianginal activity in the latter publication moreover the structure of the compounds also being different.

In formula I, halogen means fluoro, chloro, bromo or iodo.

A straight chain $C_{1-8}$ alkyl group is, for example, a methyl, ethyl, n-propyl, isopropyl, n-butyl, pentyl, hexyl, heptyl or octyl group.

A $C_{3-7}$ cycloalkyl group is a cyclopropyl, cyclobutyl, cyclopentyl, cyclohexyl or cycloheptyl group.

Preferably the halogen atom which may be represented by R is chlorine or bromine, particularly the former one.

Furthermore it is preferred that the $C_{1-8}$ alkyl group(s) which may be represented by $R_2$ and/or $R_3$ is/are such having from 1 to 6, particularly 3 to 6, carbon atoms.

It is also preferred that the $C_{3-7}$ cycloalkyl group which may be represented by $R_3$ is such having from 5 to 7, particularly 5 or 6, most particularly 6, carbon atoms.

Particularly the 2-[phenylmethylene]-1-[3'-(amino)-2'-(hydroxy)-propoxy-imino]-cyclohexane derivatives are such ones
in which

R is as above defined,

$R_2$ represents a straight chain $C_{1-6}$ alkyl group and

$R_3$ means a hydrogen atom, a straight chain $C_{1-6}$ alkyl group or a cyclohexyl group.

Compounds most particularly preferably used according to the invention are (R,S)-2-(E)-[phenylmethylene]-1-(E)-[3'-(hexylamino)-2'-(hydroxy)-propoxy-imino]-cyclohexane, (R,S)-2-(E)-[4'-(chloro)-phenylmethylene]-1-(E)-[3"-{N-methyl)-N-(cyclohexyl)-amino}-2"-(hydroxy)-propoxy-imino]-cyclohexane, (R,S)-2-(E)-[4'-(chloro)-phenylmethylene]-1-(E)-[3"-(cyclohexylamino)-2"-(hydroxy)-propoxy-imino]-cyclohexane, (R,S)-2-(E)-[4'-(chloro)-phenylmethylene]-1-(E)-[3"-(hexylamino)-2"-(hydroxy)-propoxy-imino]-cyclohexane, (R,S)-2-(E)-[4'-(chloro)-phenylmethylene]-1-(E)-[3"-(dimethylamino)-2"-(hydroxy)-propoxy-imino]-cyclohexane, (R,S)-2-(E)-[4'-(bromo)-phenylmethylene]-1-(E)-[3"-(diisopropylamino)-2"-(hydroxy)-propoxy-imino]-cyclohexane and (R,S)-2-(E)-[4'-(chloro)-phenylmethylene]-1-(E)-[(3"-(butylamino)-2"-(hydroxy)-propoxy-imino]-cyclohexane
and their stereoisomers and optical isomers and mixtures thereof as well as pharmaceutically acceptable acid addition salts and quaternary ammonium derivatives thereof.

According to a special preferred embodiment of the invention it is for preparing medicaments for the treatment of gastric and/or duodenal ulcer(s).

The salts of the compounds of formula I with pharmaceutically acceptable acids may be salts with inorganic acids, such as sulfuric acid, hydrogen chloride, hydrogen bromide or phosphoric acid, or salts with organic acids, such as acetic, propionic, methanesulfonic, p-toluenesulfonic, tartaric, succinic, maleic, fumaric, citric, malic or lactic acid, commonly used in the pharmaceutical technique.

The 2-[phenylmethylenel]-1-[3'-(amino)-2'-(hydroxy)-propoxy-imino]-cyclohexane derivatives of formula I can be prepared by using the process described in GB patent 2,235,198. Essentially, the known process comprises

a) reacting 2-[phenylmethylene]-cyclohexane-1-ones of formula

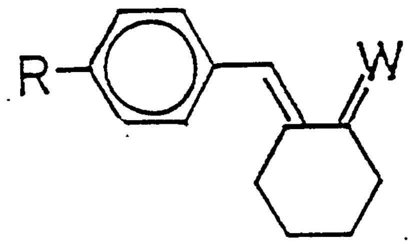

II ,

wherein W represents oxygen and R stands for a hydrogen or halogen atom, with 3-(amino)-2-(hydroxy)-propyl-hydroxylamines of formula

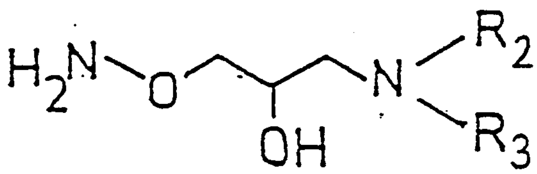

III ,

wherein

$R_2$ represents a straight chain $C_{1-8}$ alkyl group and

$R_3$ means a hydrogen atom, a straight chain $C_{1-8}$ alkyl group or a $C_{3-7}$ cycloalkyl group,

or acid addition salts thereof, or

b) reacting 2-[phenylmethylene]-cyclohexane-1-oximes of formula II, wherein W stands for a group of the formula =N-OH and R is as defined under a), with 1-(halo)-2,3-(epoxy)-propanes and then aminating the thus-obtained 2-[phenylmethylene]-1-[2',3'-(epoxy)-propoxy-imino]-cyclohexane derivatives of formula

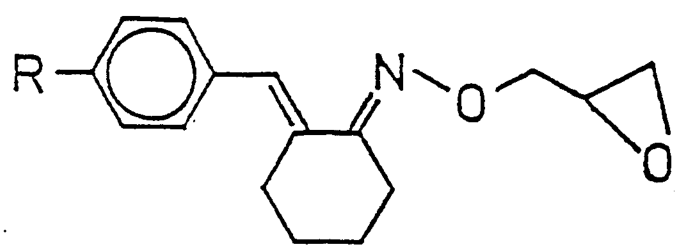

IV ,

wherein R is as defined under a), with organic bases of formula

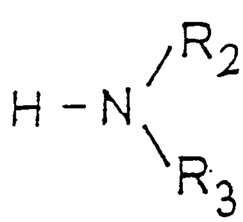

V ,

wherein $R_2$ and $R_3$ are as defined under a),

and if desired, converting 2-[phenylmethylene]-1-[3'-(amino)-2'-(hydroxy)-propoxy-imino]-cyclohexane derivatives of formula I to acid addition salts or quaternary ammonium derivatives or, if desired, liberating free 2-[phenylmethylene]-1-[3'-(amino)-2'-(hydroxy)-propoxy-imino]-cyclohexane derivative bases from salts thereof and, if desired, converting them to other salts and/or, if desired, separating stereoisomers and/or optically active isomers.

The activity of the above compounds to be used according to the invention as active ingredients for preparing medicaments having gastroprotective and antimicrobial including antiulcer activity was proved by the following tests.

1. Acute toxicity

The acute toxicity was determined on NMRI strain mice of both sexes weighing 20 to 25 g each by using 6 animals for each dose. The compounds to be tested were administered perorally in a volume of 20 ml/kg, the highest dose applied was 1 000 mg/kg. After the administration the animals were kept under usual laboratory conditions for 7 days. The $LD_{50}$ values were calculated according to the method of Litchfield and Wilcoxon (J. Pharmacol. Exp. Ther., 96 [1949], 99). The results obtained are shown in Table I.

Table I

| Compound tested | $LD_{50}$ p.o. in mg/kg |
|---|---|
| (R,S)-2-(E)-[Phenylmethylene]-1-(E)-[3'-(hexylamino)-2'-(hydroxy)-propoxy-imino]-cyclohexane | higher than 1 000 |
| (R,S)-2-(E)-[4'-(Chloro)-phenylmethylene]-1-(E)-[3"-{N-(methyl)-N-(cyclohexyl)-amino}-2"-(hydroxy)-propoxy-imino]-cyclohexane | higher than 1 000 |
| (R,S)-2-(E)-[4'-(Chloro)-phenylmethylene]-1-(E)-[3"-(cyclohexylamino)-2"-(hydroxy)-propoxy-imino]-cyclohexane | higher than 1 000 |
| (R,S)-2-(E)-[4'-(Chloro)-phenylmethylene]-1-(E)-[3"-(hexylamino)-2"-(hydroxy)-propoxy-imino]-cyclohexane | higher than 1 000 |
| (R,S)-2-(E)-[4'-(Chloro)-phenylmethylene]-1-(E)-[3"-(dimethylamino)-2"-(hydroxy)-propoxy-imino]-cyclohexane | higher than 1 000 |
| (R,S)-2-(E)-[4'-(Bromo)-phenylmethylene]-1-(E)-[3"-(diisopropylamino)-2"-(hydroxy)-propoxy-imino]-cyclohexane | 1 000 |
| (R,S)-2-(E)-[4'(Chloro)-phenylmethylene]-1-(E)-[3"-(butylamino)-2"-(hydroxy)-propoxy-imino]-cyclohexane | 1 300 |
| 5-[Methoxy]-2-[{4-(methoxy)-3,5-di-(methyl)-2-pyridyl}-methyl sulfinyl]-benzimidazole [omeprazole] {reference substance} | higher than 4 000 |
| 1-[Cyano]-2-[methyl]-3-[2-{(5-(methyl)-4-imidazolyl)-methylthio}-ethyl]-guanidine[cimetidine] {reference substance} | 2 600 |
| 3-[4-{Trans-4-(aminomethyl)-cyclohexylcarbonyloxy}-phenyl]-propionic acid [cetraxate]{reference substance} | higher than 1 000 |

The data given as "higher than" a certain value mean the highest dose administered without noting any death of animals.

2. Comparison of the gastroprotective and gastric acid secretion inhibiting effects

2.1 Test with absolute ethanol

Rats of both sexes weighing 200 to 250 g, each previously starved for 24 hours, were used in this experiment. The lesions of gastric mucosa were induced by the oral administration of absolute ethanol in a dose of 1 ml/animal. After one hour following the administration, the animals were killed by an overdose of ether, the stomach was excised and the alterations of gastric mucosa were examined. The lengths of the erosions were measured and summarized (expressed in mm) for each stomach to obtain the erosion index by calculating the average value of the group. The inhibition was calculated in percentage by comparing the value of the group treated with ethanol to that of the control group treated only with the vehicle. Various doses of the compounds to be tested were administered, perorally, in a volume of 5 ml/kg using a pretreatment time of 60 minutes. Eight rats were used for each dose. (A. Robert, Gastroenterology 77

[1979], 761 to 767).

2.2. Effect on the gastric acid secretion

The influence of the compounds on the gastric acid secretion was examined on starved Wistar rats of both sexes weighing 180 to 240 g each by using Shay's method (H. Shay et al., Gastroenterology, 5 [1945], 45). The compounds to be tested were given, perorally, 3 hours before the ligation of the pylorus. Four hours after the operation, the animals were killed by an overdose of ether, the stomach was excised, the stomach content was centrifuged and the free hydrochloric acid content of the gastric juice was determined by titration in the presence of Töpfer's reagent. The percentage of inhibition and then the $ED_{50}$ values were determined by calculating average values for the groups and comparing those to the average values for the control group. The data obtained are summarized in Table II.

Table II

Gastroprotective activity

| Compound tested | Test with absolute ethanol $ED_{50}$ p.o. in mg/kg | Relative activity related to the inhibition of the free acid[x] |
|---|---|---|
| (R,S)-2-(E)-[Phenylmethylene]-1-(E)-[3'-(hexylamino)-2'-(hydroxy)-propoxy-imino]-cyclohexane | 0.8 | about 250.0 |
| (R,S)-2-(E)-[4'-(Chloro)-phenylmethylene]-1-(E)-[3"-(cyclohexylamino)-2"-(hydroxy)-propoxy-imino]-cyclohexane | 6.1 | 30.0 |
| (R,S)-2-(E)-[4'-(Chloro)-phenylmethylene]-1-(E)-[3"-(hexylamino)-2"-(hydroxy)-propoxy-imino]-cyclohexane | 4.6 | 25.0 |

Table II continued

| Compound tested | Test with absolute ethanol $ED_{50}$ p.o. in mg/kg | Relative activity related to the inhibition of the free acid[x] |
|---|---|---|
| (R,S)-2-(E)-[4'-(Chloro)-phenyl-methylene]-1-(E)-[3"-(dimethylamino)-2"-(hydroxy)-propoxy-imino]-cyclohexane | 0.8 | 60 to 125 |
| (R,S)-2-(E)-[4'-(Bromo)-phenyl-methylene]-1-(E)-[3"-(diisopropylamino)-2"-(hydroxy)-propoxy-imino]-cyclohexane | 7.0 | about 25.0 |

Table II continued

| Compound tested | Test with absolute ethanol $ED_{50}$ p.o. in mg/kg | Relative activity related to the inhibition of the free acid[x] |
|---|---|---|
| 5-[Methoxy]-2-[{4-(methoxy)-3,5-di-(methyl)-2-pyridyl}-methyl-sulfinyl]-benzimidazole [omeprazole] {reference substance} | 4.5 | 0.9 |
| 1-[Cyano]-2-[methyl]-3-[2-{(5-⟨methyl⟩-4-imidazolyl)-methylthio}-ethyl]-guanidine [cimetidine] {reference substance} | 100 to 200 | 0.3 to 0.6 |

Table II continued

| Compound tested | Test with absolute ethanol $ED_{50}$ p.o. in mg/kg | Relative activity related to the inhibition of the free acid[x] |
|---|---|---|
| 5,11-Dihydro-11-[4-(methyl)-1-piperazinyl)-acetyl]-6H-pyrido[2,3-b][1,4]benzodiazepin-6-one [pirenzepine] {reference substance} | 18.6 | 0.4 |
| The basic aluminum salt of saccharose hydrogen sulfate [sucralfate] {reference substance} | 69.0 | - |
| 3-[4-{Trans-4-(aminomethyl)-cyclohexylcarbonyloxy}-phenyl]-propionic acid [cetraxate] {reference substance} | 76.4 | higher than 4 |

x Relative activity = $ED_{50}$ value of the gastric acid inhibition related to the $ED_{50}$ value of the inhibition of the gastric erosion.

In the test with absolute ethanol which is useful for showing the gastroprotective activity, as to the effective dose range, a sharp difference can be made between the cytoprotective effect and the gastric acid inhibition, the latter resulting in the inhibition of the gastric erosion.

From Table II it can be seen that the 3 reference compounds used in the therapy of ulcer and having a mechanism of free acid inhibition, i.e. 5-[methoxy]-2-[{4-(methoxy)-3,5-di-(methyl)-2-pyridyl}-methylsulfinyl]-benzimidazole[omeprazole], 1-[cyano]-2-[methyl]-3-benzimidazole[omeprazole], 1-[cyano]-2-[methyl]-3-[2-{(5-(methyl)-4-imidazolyl)-methyithio}-ethyl]-guanidine[cimetidine]and 5,11-di-[hydro]-11-[(4-(methyl)-1-piperazinyl)-acetyl]-6H-pyrido[2,3-b][1,4]benzodiazepin-6-one[pirenzepine], exert a relatively low gastroprotective action as compared to the gastric acid inhibiting activity their relative activity amounting to only 0.3 to 0.9. This fact indicates that the above free acid inhibiting reference compounds protect the gastric mucosa against absolute ethanol as a consequence of their gastric acid inhibiting action.

As to the 2-[phenylmethylene]-1-[3'-(amino)-2'-(hydroxy)-propoxy-imino]-cyclohexane derivates used according to the invention tested, their relative activity is very high compared to that of the reference compounds, a fact proving that the cytoprotective effect of the 2-[phenylmethylene]-1-[3'-(amino)-2'-(hydroxy)-propoxy-imino]-cyclohexane derivates used according to the invention which appears in the protection of the gastric mucosa, is independent of the acid inhibition activity.

Both reference compounds having gastroprotective effect, i.e. the basic aluminum salt of saccharose hydrogen sulfate [sucralfate] and 3-[4-{trans-4-(aminomethyl)-cyclohexylcarbonyloxy}-phenyl]-propionic acid [cetraxate], exert nearly identical low activity in the test with absolute ethanol, whereas the 2-[phenylmethylene]-1-[3'-(amino)-2'-(hydroxy)-propoxy-imino]-cyclohexane derivates used according to the invention are effective in doses lower by 1 or 2 order(s).

2.3. Test with ethanol containing hydrochloric acid

The principle of the method consists in that, simultaneously with the administration of absolute ethanol, hydrochloric acid is introduced to the stomach. Thus, the influence on systemic gastric acid inhibition action can be eliminated by the presence of the exogenous acid.

The reagent used in this test was a mixture of concentrated aqueous hydrochloric acid and absolute ethanol in a weight ratio of 1 to 50 in accordance with the dilution ratio used by Yamazaki et al. (Japan J. Pharmacol., 55 [1991], 415 to 424). In other respects, the experiment was performed as described under test 2.1., however, in addition to the erosion index calculated from the lenghts of the erosions in mm, the ratio of occurence of animals showing at least one gastric erosion within a test group, i.e. the erosion frequency, was also evaluated. The results obtained are shown in Table III.

In Table III, in case of the inhibition of the erosion frequency, the activity of the compounds tested were related to that of 3-[4-{trans-4-(aminomethyl)-cyclohexylcarbonyloxy}-phenyl]-propionic acid [cetraxate] to obtain the relative activity.

In case of the inhibition of the erosion frequency and of the erosion index, the respective $ED_{50}$ value of 3-[4-{trans-4-(aminomethyl)-cyclohexylcarbonyloxy}-phenyl]-propionic acid [cetraxate] was divided by the $ED_{50}$ value of the compound tested each to obtain the respective relative activity.

Table III

| Compound tested | Inhibition of the erosion frequency | | Inhibition of the erosion index | |
|---|---|---|---|---|
| | $ED_{50}$ p.o. in mg/kg | Relative activity | $ED_{50}$ p.o. in mg/kg | Relative activity |
| (R,S)-2-(E)-[Phenylmethylene]-1-(E)-[3'-(hexylamino)-2'-(hydroxy)-propoxy-imino]-cyclohexane | 25.7 | 7.4 | 11.5 | 1.9 |
| (R,S)-2-(E)-[4'-(Chloro)-phenylmethylene]-1-(E)-[3"-(cyclohexylamino)-2"-(hydroxy)-propoxy-imino]-cyclohexane | 30 to 60 | 3.2 to 6.3 | 10.3 | 2.1 |
| (R,S)-2-(E)-[4'-(Chloro)-phenylmethylene]-1-(E)-[3"-hexylamino)-2"-(hydroxy)-propoxy-imino]-cyclohexane | 25.2 | 7.5 | 7.2 | 3.1 |
| (R,S)-2-(E)-[4'-(Chloro)-phenylmethylene]-1-(E)-[3"-(dimethylamino)-2"-(hydroxy)-propoxy-imino]-cyclohexane | 30 to 60 | 3.2 to 6.3 | 15.8 | 1.4 |
| (R,S)-2-(E)-[4'-(Bromo)-phenylmethylene]-1-(E)-[3"-(diisopropylamino)-2"-(hydroxy)-propoxy-imino]-cyclohexane | 19.1 | 9.9 | 5.9 | 3.7 |
| 3-[4-{Trans-4-(aminomethyl)-cyclohexylcarbonyloxy}-phenyl]-propionic acid[cetraxate] {reference substance} | 189.0 | 1.0 | 22.0 | 1.0 |

From Table III it is to be taken that the direct cytoprotective activity (considering both the inhibition of the erosion frequency and the inhibition of the erosion index) of the compounds used according to the invention is substantially higher than that of the 3-[4-{trans-4-(aminomethyl)-cyclohexylcarbonyloxy}-phenyl]-propionic acid [cetraxate] used as reference compound, although the number of lesions in the gastric mucosa was increased and the lesions became more severe due to the presence of hydrochloric acid in the ethanol.

In the test according to Shay (section 2.2.), only a moderate gastric acid secretion inhibition was shown by the-compounds according to the invention as well as by 3-[4-{trans-4-(aminomethyl)-cyclohexylcarbonyloxy}-phenyl]-propionic acid [cetraxate] having cytoprotective activity. In the above test (section 2.3.), the high activity of the compounds used according to the invention in the presence of exogenous hydrochloric acid precludes the possibility that the acid inhibition effect would be predominant in the gastroprotective activity of the compounds. Simultaneously this test demonstrates the superiority of the compounds used according to the invention as compared to 3-[4-{trans-4-(aminomethyl)-cyclohexylcarbonyloxy}-phenyl]-propionic acid [cetraxate] as regards the cytoprotective activity.

3. Antibacterial effect on Helicobacter pylori

Helicobacter pylori strains used in these experiments were obtained from the biopsial samples of the stomachs of patients suffering from ulcerative diseases of the gastrointestinal system. From the pure culture of isolates a thick suspension was prepared with cysteine-inositol solution suitable for lyophilisation, and the suspension obtained was maintained in cold-resistant plastic ampoules in a deep-freezing chamber at a temperature of about -196°C. For the determination of the inhibitory effect of the compounds tested on Helicobacter pylori, blood agar containing 10 mg/litre of a stereoisomer of 23-[aminocarbonyl]-12-[{2-0-(3-amino-2,3,6-trideoxy-3-C-methyl-α-L-lyxo-hexopyranosyl)-β-D-glucopyranosyl}-oxy]-8,16-di-[chloro]-2,3,4,5,19,20,21,22,23,24,25,26,27,28,29,30-hexa-[decahydro]-5,19,34,36,38-penta-[hydroxy]-28-[{4-(methyl)-2-(methylamino)-1-(oxo)-pentyl}-amino]-3,21,25,28,41-penta-[oxo]-1H-6,9:15,18-[dietheno]-4,30-[iminomethano]-31,35-[metheno]-11,27,13-[[1]-propene[1,2]diyl[3]ylidene]-13H-10,14,2,22,26,29-benzodioxatetraazacycloheptatriacontine-1-carboxylic acid ⟨vancomycin⟩ and 25 mg/litre of [1R-(1R$^x$,3S$^x$,5R$^x$,6R$^x$,9R$^x$,11R$^x$,15S$^x$,16R$^x$,17R$^x$,18S$^x$,19E,21E,23E,25E,27E,29E,31E,33R$^x$,35S$^x$,36R$^x$,37S$^x$)]-33-[{3-(amino)-3,6-di-(deoxy)-β-D-mannopyranosyl}-oxy]-1,3,5,6,9,11,17,37-octa-[hydroxy]-15,16,18-tri-[methyl]-13-[oxo]-14,39-di-[oxa]-bicyclo-[33.3.1]-nonatriaconta-19,21,23,25,27,29,31-heptaene-36-carboxylic acid ⟨amphotericin B⟩ was used (C. A. M. McNulty and I. C. Dent: Susceptibility of clinical isolates of Campylobacter pylori to twenty one antimicrobial agents. Book of Abstracts, 4th European Congress of Clinical Microbiology, Nice [1989]).

The plates containing the strains were cultivated at 37°C for 72 hours under a gaseous atmosphere containing 10% of carbon dioxide and 5% of oxygen (D. M. Jones et al., J. Clin. Pathol., 37 [1984], 1 002).

The culture media were inoculated with a dilution of the broth culture of the strains grown overnight or with a suspension prepared from a culture obtained on a solid medium. About $10^5$ of germs were applied to the surface of each plate.

A series of dilution were prepared from the compound to be tested. The MIC (minimal inhibiting concentration) value of the compounds tested was considered to be the lowest concentration inhibiting the growth of the test organism completely. The results obtained for (R,S)-2-(E)-[4'-(chloro)-phenylmethylene]-1-(E)-[3"-(hexylamino)-2"-(hydroxy)-propoxy-imino]-cyclohexane and colloidal bismuth subcitrate used as reference are given in Table IVa.

Table IVa

| Helicobacter pylori strains | MIC value in microgram/ml of | |
|---|---|---|
| | (R,S)-2-(E)-[4'-(chloro)-phenylmethylene]-1-(E)-[3"-(hexylamino)-2"-(hydroxy)-propoxy-imino]-cyclohexane | Bismuth subcitrate [reference compound] |
| 867/90 | 1.9 | |
| 1596 | 0.9 | |
| 3842 | 1.9 | 31.2 to 62.5$^x$ |
| 6656 | 1.9 | |
| 6709 | 1.9 | |

$^x$Results relating to a Helicobacter pylori strain isolated from 7 biopsial samples.

From Table IVa it can be seen that (R,S)-2-(E)-[4'-(chloro)-phenylmethylene]-1-(E)-[3"-(hexylamino)-2"-(hydroxy)-propoxy-imino]-cyclohexane was substantially more effective in the test than the colloidal bismuth subcitrate widely used in the therapy of Helicobacter pylori infections. The activity of (R,S)-2-(E)-[4'-(chloro)-phenylmethylene]-1-(E)-[3"-(hexylamino)-2"-(hydroxy)-propoxy-imino]-cyclohexane stands near to that of antibiotics (Y. Glupczynski: In vitro susceptibility testing of Helicobacter pylori and pharmacology of antimicrobial agents. Abstract book, Workshop Helicobacter pylori and the new concepts in gastro-duodenal diseases, Prague [1992]).

Thus the 2-[phenylmethylene]-1-[3'-(amino)-2'-(hydroxy)-propoxy-imino]-cyclohexane derivatives used according to the invention possess excellent cytoprotective and antimicrobial effects. Consequently, they can be advantageously used for the treatment of gastrointestianl diseases, where the gastrointestinal mucosa is injured or the weakening of the protective factors plays a pathogenic role, e.g.:

- long-lasting treatment of rheumatoid diseases with non-steroidal antiinflammatory drugs,
- ulcers, especially duodenal ulcers, which cannot be treated with antiulcer drugs which inhibit gastric acid,
- chronic erosive gastritis (wherein the acid secretion is within the normal limits) and
- bacterial infections caused, in the first place, by Helicobacter pylori playing a significant role in the relapse of gastroduodenal diseases.

The known weak gastric acid secretion inhibiting activity of the 2-[phenylmethylene]-1-[3'-(amino)-2'-(hydroxy)-propoxy-imino]-cyclohexane derivatives used according to the invention does not play any essential role in their valuable

novel gastroprotective effect.

The medicaments for the preparation of which the 2-[phenylmethylene]-1-[3'-(amino)-2'-(hydroxy)-propoxy-imino]-cyclohexane derivatives of formula I and their stereoisomers and optical isomers and mixtures thereof as well as pharmaceutically acceptable acid addition salts and quaternary ammonium derivatives thereof are used can be pharmaceutical compositions. They can be prepared by admixing the active ingredient(s) to 1 or more pharmaceutically acceptable carrier(s), and converting the mixture obtained to a pharmaceutical composition by known methods of the drug manufacture. As to the additives and methods see e.g. Remington's pharmaceutical Sciences, 18th Edition, Mack Publishing Company, Easton, USA.

The use according to the invention may be such for preparing medicaments for oral, rectal, parenteral or local administration.

For oral administration, for example, powders, tablets, coated tablets, dragées and capsules may be prepared. These dosage units contain, in general, 10 to 100 mg of active ingredient in addition to 1 or more carrier(s) which may be e.g. binding agents, such as gelatine, sorbitol and/or poly-(vinylpyrrolidone), filling agents, such as lactose, glucose, starch and/or calcium phospate, and/or auxiliary substances for tabletting, such as magnesium stearate, talc, poly-(ethylene glycol) and/or silica. By the use according to the invention as liquid pharmaceutical compositions for oral administration preferably aqueous suspensions and/or elixirs are prepared. The preparation can be carried out by using solvents or diluents, such as water, ethanol, propylene glycol and/or glycerol, suspending agents, such as gelatine and/or carboxymethylcellulose, taste improving agents, such as sorbitol and/or sugar solution, preservatives, such as methyl p-hydroxybenzoate, and/or dyes.

By the use according to the invention as pharmaceutical compositions suitable for parenteral administration sterile solutions of the active ingredient(s) may be prepared.

The pharmaceutical compositions prepared by the use according to the invention contain, in general, 0.1 to 95.0 per cent of the active substance(s). The medicaments prepared by the use according to the invention can be administered in an effective non-toxic dose of [a] 2-[phenylmethylene]-1-[3'-(amino)-2'-(hydroxy)-propoxy-imino]-cyclohexane derivative(s) used according to the invention to patients suffering from gastrointestinal disorders.
A typical dose for adult patients amounts to 0.25 to 40 mg/kg, preferably 1 to 20 mg/kg, of the 2-[phenylmethylene]-1-[3'-(amino)-2'-(hydroxy)-propoxy-imino]-cyclohexane derivative(s) used according to the invention, in a single daily dose or in 2 to 3 subdoses. In each case, the dose to be administered depends on the activity of the active ingredient(s) used, route of administration, condition of the patient to be treated and other factors.

The invention is illustrated in detail by the following Examples.

Example 1

| Preparation of tablets | |
|---|---|
| Composition | Amount in mg/tablet |
| Active ingredient(s) 2-[phenylmethylene]-1-[3'-(amino)-2'-(hydroxy)-propoxy-imino]-cyclohexane derivative(s)(used according to the invention) | 25.0 |
| Corn starch | 97.0 |
| Poly-(vinylpyrrolidone) | 175.0 |
| Magnesium stearate | 3.0 |
| | 300.0 |

The active ingredient(s) and corn starch are wetted with a 15% by weight aqueous poly-(vinylpyrrolidone) solution, then granulated, and the wet granules are dried at 40 to 45°C. The dried granules are thoroughly mixed with magnesium stearate, and the mixture is compressed to obtain tablets weighing 300.0 mg each.

Example 2

| Preparation of dragées | |
|---|---|
| Composition | Amount in mg/dragée |
| Active ingredient(s) 2-[phenylmethylene]-1-[3'-(amino)-2'-(hydroxy)k-propoxy-imino]-cyclohexane derivative(s) (used according to the invention) | 50.0 |
| Lactose | 94.0 |
| Magnesium stearate | 2.0 |
| Poly-(vinylpyrrolidone) | 4.0 |
| | 150.0 |

Granules are prepared as described in Example 1, however, the granulation is carried out with a mixture of the active ingredient(s) and lactose (instead of corn starch), then the granules are compressed to obtain tablets weighing 150 mg each. The tablets obtained are used as dragée-cores which are coated with a layer consisting of talc and sugar in a manner known per se. The coated tablets are coloured with a food dye and polished with bee-wax.

Example 3

| Preparation of capsules | |
|---|---|
| Composition | Amount in mg/capsule |
| Active ingredient(s) 2-[phenylmethylene]-1-[3'-(amino)-2'-(hydroxy)k-propoxy-imino]-cyclohexane derivative(s) (used according to the invention) | 25.0 |
| Corn starch | 122.0 |
| Colloidal silica | 3.0 |
| | 150.0 |

The ingredients listed are homogenized and the mixture obtained is filled into hard gelatine capsules. Each capsule contains 150 mg of the powder mixture.

## Claims

1. The use of 2-[phenylmethylene]-1-[3'-(amino)-2'-(hydroxy)-propoxy-imino]-cyclohexane derivatives of formula

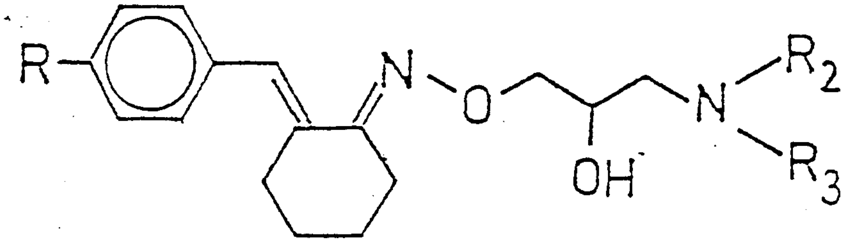

I ,

wherein

R means a hydrogen or halogen atom,

$R_2$ represents a straight chain $C_{1-8}$ alkyl group and

$R_3$ means a hydrogen atom, a straight chain $C_{1-8}$ alkyl group or a $C_{3-7}$ cycloalkyl group,

and their stereoisomers and optical isomers and mixtures thereof as well as pharmaceutically acceptable acid addition salts and quaternary ammonium derivatives thereof, optionally in admixture with 1 or more carrier(s) commonly used in pharmaceutical compositions, for preparing medicaments having combined gastroprotective and antimicrobial including antiulcer activity.

2. The use according to claim 1, characterized in that the halogen atom which may be represented by R is chlorine.

3. The use according to claim 1 or 2, characterized in that the $C_{1-8}$ alkyl group(s) which may be represented by $R_2$ and/or $R_3$ is/are such having from 1 to 6, particularly 3 to 6, carbon atoms.

4. The use according to claims 1 to 3, characterized in that the $C_{3-7}$ cycloalkyl group which may be represented by $R_3$ is such having from 5 to 7, particularly 5 or 6, carbon atoms.

5. The use according to claims 1 to 4, characterized in that the 2-[phenylmethylene]-1-[3'-(amino)-2'-(hydroxy)-propoxy-imino]-cyclohexane derivatives are (R,S)-2-(E)-[phenylmethylene]-1-(E)-[3'-(hexylamino)-2'-(hydroxy)-propoxy-imino]-cyclohexane, (R,S)-2-(E)-[4'-(chloro)-phenylmethylene]-1-(E)-[3''-{N-(methyl)-N-(cyclohexyl)-amino}-2''-(hydroxy)-propoxy-imino]-cyclohexane, (R,S)-2-(E)-[4'-(chloro)-phenylmethylene]-1-(E)-[3''-(cyclohexylamino)-2''-(hydroxy)-propoxy-imino]-cyclohexane, (R,S)-2-(E)-[4'-(chloro)-phenylmethylene]-1-(E)-[3''-(hexylamino)-2''-(hydroxy)-propoxy-imino]-cyclohexane, (R,S)-2-(E)-[4'-(chloro)-phenylmethylene]-1-(E)-[3''-(dimethylamino)-2''-(hydroxy)-propoxy-imino]-cyclohexane, (R,S)-2-(E)-[4'-(bromo)-phenylmethylene]-1-(E)-[3''-(diisopropylamino)-2''-(hydroxy)-propoxy-imino]-cyclohexane and (R,S)-2-(E)-[4'-(chloro)-phenylmethylene]-1-(E)-[3''-(butylamino)-2''-(hydroxy)-propoxy-imino]-cyclohexane and their stereoisomers and optical isomers and mixtures thereof as well as pharmaceutically acceptable acid addition salts and quaternary ammonium derivatives thereof.

6. The use according to claims 1 to 5, characterized in that it is for preparing medicaments for oral, rectal, parenteral or local administration.

7. The use according to claims 1 to 6,characterized in that it is for preparing medicaments for the treatment of gastric and/or duodenal ulcer(s).

**Patentansprüche**

1. Verwendung von 2-[Phenylmethylen]-1-[3'-(amino)-2'-(hydroxy)-propoxy-imino]-cyclohexan-Derivaten der Formel

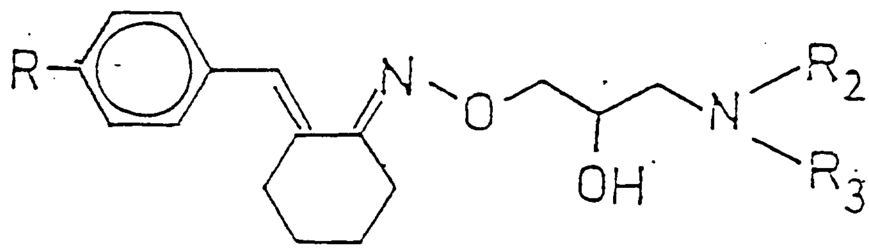

I ,

worin

R ein Wasserstoff- oder Halogenatom bedeutet,
$R_2$ für eine geradkettige $C_{1-8}$-Alkylgruppe steht und
$R_3$ ein Wasserstoffatom, eine geradkettige $C_{1-8}$-Alkylgruppe oder eine $C_{3-7}$-Cycloalkygruppe bedeutet,

und deren Stereoisomere und optische Isomere und Mischungen davon sowie pharmazeutisch annehmbare Säureadditionssalze und quaternäre Ammoniumderivate davon, gegebenenfalls in Vermischung mit einem oder mehreren Trägern, welche üblicherweise in pharmazeutischen Zusammensetzungen verwendet werden, zur Herstellung von Medikamenten mit kombinierter gastroprotektiver und antimikrobieller Aktivität, einschließlich Antiulcusaktivität.

**2.** Verwendung gemäß Anspruch 1, dadurch gekennzeichnet, daß das Halogenatom, welches durch R repräsentiert sein kann, Chlor ist.

**3.** Verwendung gemäß Anspruch 1 oder 2, dadurch gekennzeichnet, daß die $C_{1-8}$-Alkylgruppe(n), welche durch $R_2$ und/oder $R_3$ repräsentiert sein können, eine solche ist bzw. solche sind mit 1 bis 6, insbesondere 3 bis 6, Kohlenstoffatomen.

**4.** Verwendung gemäß Anspruch 1 bis 3, dadurch gekennzeichnet, daß die $C_{3-7}$-Cycloalkylgruppe, welche durch $R_3$ repräsentiert sein kann, eine solche mit 5 bis 7, insbesondere 5 oder 6, Kolllenstoffatomen ist.

**5.** Verwendung gemäß 1 bis 4, dadurch gekennzeichnet, daß die 2-[Phenylmethylen]-1-[3'-(amino)-2'-(hydroxy)-propoxy-imino]-cyclohexan-Derivate
(R,S) -2- (E)-[Phenylmethylen]-1-(E)- [3'-(hexylamino)-2'-(hydroxy)-propoxy-imino]-cyclohexan, (R,S)-2-(E)-[4'-(Chlor)-phenylmethylen]-1-(E)-[3"-{N-(methyl)-N-(cyclohexyl)-amino}-2"-(hydroxy)-propoxy-imino]-cyclohexan, (R,S)-2-(E)-[4'-(Chlor)-phenylmethylen]-1-(E)-[3"-(cyclohexylamino)-2"-(hydroxy)-propoxy-imino]-cyclohexan, (R,S)-2-(E)-[4'-(Chlor)-phenylmethylen]-1-(E)-[3"-(hexylamino)-2"-(hydroxy)-propoxy-imino]-cyclohexan, (R,S)-2-(E)-[4'-(Chlor)-phenylmethylen]-1-(E)-[3"-(dimethylamino)-2"-(hydroxy)-propoxy-imino]-cyclohexan, (R,S)-2-(E)-[4'-(Brom)-phenylmethylen]-1-(E)-[3"-(diisopropylamino)-2"-(hydroxy)-propoxy-imino]-cyclohexan und (R,S)-2-(E)-[4'-(Chlor)-phenylmethylen]-1-(E)-[3"-(butylamino)-2"-(hydroxy)-propoxy-imino]-cyclohexan
und ihre Stereoisomeren und optischen Isomeren und Mischungen davon sowie pharmazeutisch annehmbare Säureadditionssalze und quaternäre Ammoniumderivate davon sind.

**6.** Verwendung gemäß Anspruch 1 bis 5, dadurch gekennzeichnet, daß sie für die Herstellung von Medikamenten zur oralen, rektalen, parenteralen oder lokalen Verabreichung ist.

**7.** Verwendung gemäß Anspruch 1 bis 6, dadurch gekennzeichnet, daß sie zur Herstellung von Medikamenten für die Behandlung von Magen- und/oder Duodenalgeschwür ist.

**Revendications**

**1.** L'utilisation de dérivés du 2-[phénylméthylène)-1-(3'-(amino)-2'-(hydroxy)-propoxy-imino]-cyclohexane de formule

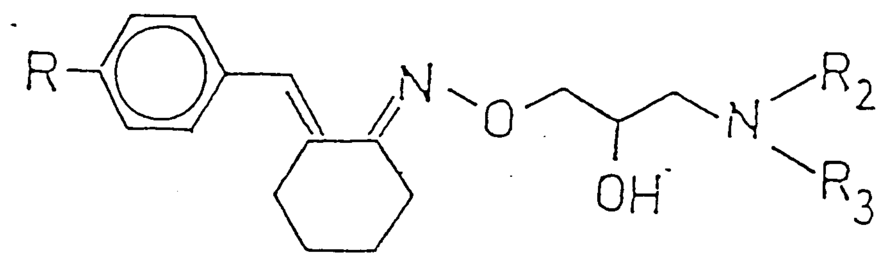

I,

dans laquelle

R signifie un atome d'hydrogène ou d'halogène,
$R_2$ représente un groupe alkyle en $C_{1-8}$ à chaîne droite, et
$R_3$ signifie un atome d'hydrogène, un groupe alkyle en $C_{1-8}$ à chaîne droite ou un groupe cycloalkyle en $C_{3-7}$,

et leurs stéréoisomères et isomères optiques ainsi que des mélanges de ceux-ci, de même que des sels d'addition d'acide pharmaceutiquement acceptables et des dérivés ammonium quaternaire de ceux-ci, le cas échéant mélangés à un ou plusieurs véhicules communément utilisés dans des compositions pharmaceutiques,

afin de préparer des médicaments combinant une activité gastroprotectrice et antimicrobienne, y compris une activité antiulcéreuse.

2. L'utilisation selon la revendication 1, caractérisée en ce que l'atome d'halogène qui peut être représenté par R est le chlore.

3. L'utilisation selon la revendication 1 ou la revendication 2, caractérisée en ce que le ou les groupes alkyles en $C_{1-8}$, qui peuvent être représentés par $R_2$ et/ou $R_3$ est/sont de préférence tels qu'ils a/ont de 1 à 6, de préférence de 3 à 6 atomes de carbone.

4. L'utilisation selon les revendications 1 à 3, caractérisée en ce que le groupe cycloalkyle en $C_{3-7}$, qui peut être représenté par $R_3$ possède de 5 à 7, en particulier 5 ou 6, et plus particulièrement 6 atomes de carbone.

5. L'utilisation selon les revendications 1 à 4, caractérisée en ce que les dérivés de 2-[phénylméthylène]-1-[3'-(amino)-2'-(hydroxy)-propoxy-imino]-cyclohexane sont les suivants: sont les suivants:

   (R,S)-2-(E)-[phénylméthylène]-1-(E)-[3'-(hexylamino)-2'-(hydroxy)-propoxy-imino]-cyclohexane,
   (R,S)-2-(E)-[4'-(chloro)-phénylméthylène]-1-(E)-[3"-{(N-méthyl)-N-(cyclohexyl)-amino}-2"-(hydroxy)-propoxy-imino]-cyclohexane,
   (R,S)-2-(E)-[4'-(chloro)-phénylméthylène]-1-(E)-[3"-(cyclohexylamino)-2"-(hydroxy)-propoxy-imino]-cyclohexane,
   (R,S)-2-(E)-[4'-(chloro)-phénylméthylène]-1-(E)-[3"-(hexylamino)-2"-(hydroxy)-propoxy-imino]-cyclohexane,
   (R,S)-2-(E)-[4'-(chloro)-phénylméthylène]-1-(E)-[3"-(diméthylamino)-2"-(hydroxy)-propoxy-imino]-cyclohexane,
   (R,S)-2-(E)-[4'-(bromo)-phénylméthylène]-1-(E)-[3"-(diisopropylamino)-2"-(hydroxy)-propoxy-imino]-cyclohexane, et
   (R,S)-2-(E)-[4'-(chloro)-phénylméthylène]-1-(E)-[3"-(butylamino)-2"-(hydroxy)-propoxy-imino]-cyclohexane,

   ainsi que leurs stéréoisomères et isomères optiques et des mélanges de ceux-ci, de même que leurs sels d'addition d'acide pharmaceutiquement acceptables et leurs dérivés d'ammonium quaternaire de ceux-ci.

6. L'utilisation selon les revendications 1 à 5, caractérisée en ce qu'elle est destinée à la préparation de médicaments pour administration par voie orale, rectale, parentérale ou locale.

7. L'utilisation selon les revendications 1 à 6, caractérisée en ce qu'elle est destinée à la préparation de médicaments pour le traitement des ulcères de l'estomac et/ou du duodénum.